# EUROPEAN PATENT APPLICATION

(11) **EP 4 768 596 A1**
(43) Date of publication of application: **01.07.2026**
(21) Application number: 25188180.1
(22) Date of filing: 08.07.2025
(51) Int. Cl.: C12Q 1/6804, C12Q 1/6816

(54) **LABEL, MARKER AND METHOD FOR ANALYSING BIOLOGICAL SAMPLES**

(30) Priority: 30.12.2024 EP 24223671
(71) Applicant: Leica Microsystems CMS GmbH, 35578 Wetzlar (DE)
(72) Inventor: Alsheimer, Soeren, 35578 Wetzlar (DE)
(74) Representative: Schaumburg und Partner Patentanwälte mbB

(57) **Abstract**

A label for analysing a biological sample is provided. The label comprises a first label part comprising a first nucleic acid strand (106) and a second label part comprising a second nucleic acid strand (110). The first nucleic acid strand (106) and the second nucleic acid strand (110) are configured to form a duplex. The label further comprises at least one first labelling moiety (108) and at least one second labelling moiety (112), and the label further comprises at least one blocking nucleic acid strand (126, 128). In further aspects, a marker (100, 500, 600, 700, 1000) comprising the label and a respective method are provided.

## Description

### Technical field

The invention relates to a label for analysing biological samples comprising at least one blocking nucleic acid strand. In a further aspect, a marker and a method for analysing biological samples is provided.

### Background

Labels and markers are frequently used when analysing biological samples, for example in fluorescent microscopy. Markers for fluorescent microscopy generally comprise affinity reagents, such as antibodies, and labels attached to the affinity reagents, in order to enable specifically attaching the labels - in particular via the affinity reagents - to a target analyte in a biological sample. This allows the identification and/or localisation of the target analyte in the biological sample.

The various applications of labels and markers include multiplexing approaches, which require large sets of distinguishable labels and markers, whilst detecting analytes that only occur in small quantities requires markers with bright labels. In particular when handling large sets of markers, which may be assembled using labels and from individual components such as dyes and affinity reagents, it is of interest to reduce complexity of handling whilst maintaining flexibility of use and reliability.

### Summary

It is an object to provide a label and a marker that enables efficient handling when analysing biological samples.

The aforementioned object is achieved by the subject-matter of the independent claims. Advantageous embodiments are defined in the dependent claims and the following description.

In a first aspect, a label for analysing a biological sample is provided. the label comprises a first label part comprising a first nucleic acid strand and a second label part comprising a second nucleic acid strand. The first nucleic acid strand and the second nucleic acid strand are configured to form a duplex. The label further comprises at least one first labelling moiety and at least one second labelling moiety. The label further comprises at least one blocking nucleic acid strand at least partially complementary to one of the first nucleic acid strand and the second nucleic acid strand.

The blocking nucleic acid strand prevents undesired or uncontrolled hybridisation between the first and second label parts. In turn, this enables efficiently handling of the label parts, for example, when preparing to analyse a biological sample. For example, when using the label to analyse the proximity between target analytes of a biological sample.

The label is preferably configured to be optically detectable at least when the first label part and second label part are in close proximity. In particular, the labelling moieties are (covalently) attached to one of the first and second nucleic acid strands. For example, the first and second labelling moieties may be a fluorescence resonance energy transfer (FRET) pair, which is optically detectable when they are in close proximity, in particular within 1 to 10 nm. This may be used for FRET-based proximity assays, in which the FRET efficiency between different label parts of a marker may be measured by performing readouts in the presence and absence of blocking strand(s), i.e. before and after the removal of blocking strands. FRET may be measured by reading intensities and/or changes in fluorescence lifetime, for example by means of a confocal microscope.

In particular, the first nucleic acid strand may comprise a first nucleotide sequence complementary to a second nucleotide sequence of the second nucleic acid strand. Preferably, the first nucleotide sequence and the second nucleotide sequence are at least partially complementary to each other, in particular based on Watson-Crick base pairing. Thus, the first and the second sequence may form the duplex structure with each other. This enables stable hybridisation of the first nucleotide sequence and the second nucleotide sequence to each other. The duplex may be a double helix, such as B-form DNA.

In an embodiment, the label comprises a (first) plurality of the blocking nucleic acid strands, wherein the blocking nucleic acid strands of the plurality of blocking nucleic acid strands are linked to each other by cleavable linkers, in particular in order to form a cleavable linear chain of the blocking nucleic acid strands. In particular, the cleavable linkers may be selectively cleavable by applying a cleavage agent. This enables efficiently and selectively removing the blocking nucleic acid strands. Generally, the plurality of blocking nucleic acid strands linked by the cleavable linkers form a more thermodynamically stable duplex with the first or second nucleic acid strand than the shorter, individual blocking nucleic acid strands after they have been cleaved from each other. In particular, the individual blocking nucleic acid strands have a lower melting temperature or a decreased magnitude of the free energy of hybridisation (ΔG), resulting in lower hybridisation stability, which enables easily removing the individual blocking nucleic acid strands from the first or second nucleic acid strand.

In an embodiment, the cleavable linkers are photocleavable linkers, chemically cleavable linkers, or enzymatically cleavable linkers. This enables specifically cleaving the linkers, in particular, without affecting the remaining elements of the label. In a particular embodiment, the cleavable linkers are not nucleotides or nucleic acids, or do not comprise nucleotides or nucleic acids.

Photocleavable linkers may be cleavable by irradiation with (UV) light as the cleavage agent, for example. An example of a specific photocleavable linker is BMN linker (Bio-mers).

The chemically cleavable linker may be cleavable by contact with a specific chemical as the cleavage agent. For example, the chemically cleavable linker may comprise an aryl boronic acid ester cleavable by hydrogen peroxide, or a disulfide bond cleavable by reduction by TCEP, DTT, or glutathione. Thiazole, thiazolidine, oxime, hydrazone linkers are likewise chemically cleavable under conditions that are generally compatible with biological assays. pH-sensitive linkers such as hydrazone linkers may be cleaved at pH4-6, for example.

Enzymatically cleavable linkers may be cleavable by contact with a specific enzyme as the cleavage agent. For example, a valine-citrulline dipeptide linker is cleavable by cathepsin B. Alternatively, cleavable linkers may comprise sequence stretches that are cleaved by a naturally occurring or engineered cleaving enzyme configured to cleave only one of the strands of a DNA duplex. Examples of cleaving enzymes and their sequence stretches include Nt.BstNBI, NEB (5'-GAGTC-3'), Nb.Btsl, NEB (5'-GCAGTG-3'), Nb.BsrDl, NEB (5'-GCAATG-3'), Nt.BbvCl, NEB (5'-CCTCAGC-3'), Nt.CviPll, NEB (5'-CCD-3' (D=A, G, or T)), Nt.CviQII, NEB (5'-RAG-3' (R=A or G)), I-Hmul, NEB (Recognizes degenerate sequences, I-BasI, NEB (Recognizes degenerate sequences), Nb.Mva1269I, Thermo Fisher Scientific (5'-GAATGC-3').

In an embodiment, all the blocking nucleic acid strands of the plurality of blocking nucleic acid strands are complementary to respective nucleotide sequences of either the first nucleic acid strand or the second nucleic acid strand. This enables effectively blocking the hybridisation between the first and second nucleic acid strands. In particular, adjacent blocking nucleic acid strands of a cleavable linear chain of the blocking nucleic acid strands are complementary to respective adjacent nucleotide sequences of the first or second nucleic acid strands. Thus, the plurality of blocking nucleic acid strands of the label may each hybridise to a different part of the first nucleic acid strand or to a different part of the second nucleic acid strand.

In an embodiment, adjacently linked blocking nucleic acid strands of the plurality of blocking nucleic acid strands are complementary to respective adjacent nucleotide sequences of the first nucleic acid strand or the second nucleic acid strand. This enables a high melting temperature or an increase magnitude of the free energy of hybridisation (ΔG) for the linked blocking nucleic acid strands, resulting in high hybridisation stability. The linked blocking nucleic acid strands may form a cleavable linear chain of the blocking nucleic acid strands that is complementary to the respective nucleotide sequences along the first or second nucleic acid strands. Thus, the plurality of blocking nucleic acid strands of the label may each hybridise to a different part of the first nucleic acid strand or to a different part of the second nucleic acid strand.

In an embodiment, each of the at least one blocking nucleic acid strands, particularly, of the plurality of blocking nucleic acid strands, comprises a number of nucleotides in a range of 5 to 15 nucleotides, preferably, 5 to 12 nucleotides, more preferably, 6 to 9 nucleotides. This enables a lower melting temperature or a decreased magnitude of the free energy of hybridisation (ΔG), resulting in lower hybridisation stability, for each of the blocking nucleic acid strands individually. The nucleotides of each of the blocking nucleic acid strands may be linked to each other via phosphodiester bonds to form the respective nucleic acid strands.

Preferably, the at least one blocking strand may have a melting temperature in a range of 28-42°C, in particular between 28-36°C. The at least one blocking strand may have a mismatched sequence to its respective sequence on the first or second nucleic acid strands. Thus, the at least one blocking strand may have one or more mismatched nucleotides compared to the respective sequence on the first or second nucleic acid strands. This enables tuning the hybridisation stability of the at least one blocking strand.

In an embodiment, the first nucleic acid strand and/or the second nucleic acid strand comprise, in particular each comprise, a number of nucleotides in a range of 50 to 150 nucleotides.

In an embodiment, the at least one blocking nucleic acid strand is complementary to a part of the first nucleic acid strand or a part of the second nucleic acid strand that are complementary to each other. This enables effectively blocking the hybridisation between the first and second nucleic acid strands.

In an embodiment, the label comprises a second plurality of blocking nucleic acid strands, wherein the blocking nucleic acid strands of the second plurality of blocking nucleic acid strands are linked to each other by cleavable linkers, and wherein the blocking nucleic acid strands of the second plurality of blocking nucleic acid strands are (at least partially) complementary to respective nucleotide sequences of the second nucleic acid strand. This enables blocking both, the first and second nucleic acid strands with dedicated blocking nucleic acid strands. Preferably in this case, the blocking nucleic acid strands of the first plurality of blocking nucleic acid strands may be (at least partially) complementary to respective nucleotide sequences of the first nucleic acid strand.

The linked blocking nucleic acid strands of the second plurality may form a second cleavable linear chain of blocking nucleic acid strand. The cleavable linkers may be selectively cleavable by applying a cleavage agent. The cleavable linkers of the second plurality of blocking nucleic acid strands may be configured in a similar manner as those described for the first plurality of blocking nucleic acid strands.

In an embodiment, the at least one first labelling moiety and/or the at least one second labelling moiety is optically detectable. This enables efficient detection of the label, for example, by means of a microscope, in particular a fluorescence microscope. For example, the first labelling moiety and/or the second labelling moiety may comprise a fluorophore such as fluorescent proteins, organic or inorganic fluorescent molecules, or fluorescent nanoparticles.

In an embodiment, the at least one first labelling moiety and/or the at least one second labelling moiety have identical optical characteristics, or the at least one first labelling moiety and/or the at least one second labelling moiety have different optical characteristics. The optical characteristics may include excitation or emission wavelength, or fluorescence lifetime, for example.

In an embodiment, the at least one first labelling moiety and the at least one second labelling moiety are configured for non-radiative energy transfer between them, in particular when they are in close proximity. This enables efficiently determining whether the label parts are in close proximity to each other. For example, the first labelling moiety and the second labelling moiety may be configured to form a FRET pair with each other, where one is the FRET donor and the other is the FRET acceptor. The FRET between the first labelling moiety and the second labelling moiety may generally occur when the label parts are hybridised to each other and therefore the labelling moieties are in close proximity.

In an embodiment, the at least one first labelling moiety and the at least one second labelling moiety are both, preferably covalently, attached to either the first nucleic acid strand or the second nucleic acid strand. For example, they may be attached to the phosphate backbone of the respective nucleic acid. In particular, the labelling moiety may be attached to opposite ends of the respective first or second nucleic acid strand. For example, the at least one first labelling moiety may be attached towards a 3' end of the respective nucleic acid strand and the second labelling moiety may be attached towards a 5' end of the respective nucleic acid strand.

In an embodiment, the at least one first labelling moiety is, preferably covalently, attached to the first nucleic acid strand, for example to a phosphate backbone of the nucleic acid strand, and the at least one second labelling moiety may be (covalently) attached to the second nucleic acid strand, for example to a phosphate backbone of the nucleic acid strand. In this case, the optical properties of the first and second labelling moieties may similarly change due to their proximity when the first and second nucleic acid strands are hybridised to each other.

In an embodiment, the first nucleic acid strand extends along a first direction and a plurality of the first labelling moieties are arranged on the first nucleic acid strand along the first direction, and/or the second nucleic acid strand extends along a second direction and a plurality of the second labelling moieties are arranged on the second nucleic acid strand along the second direction. This enables generating an optically detectable signal that is proportional to the distance between the first label part and the second label part. In particular, the respective labelling moieties are arranged one after another along the respective direction. In particular, the respective labelling moieties are arranged one after another.

In an embodiment, the label comprises a third label part comprising a third nucleic acid strand, wherein the third nucleic acid strand is configured to form a triplex with the first nucleic acid strand and the second nucleic acid strand. Preferably, the labelling moieties are (only) arranged on or attached to the third nucleic acid strand and not on the other nucleic acid strands.

In particular in case the label comprises the third label part, the label may comprise a third blocking nucleic acid strand at least partially complementary to the third nucleic acid strand. The third nucleic acid strand and the third blocking nucleic acid strand may additionally be supplemented with features that are described for the first nucleic acid strand or the second nucleic acid strand, or the first blocking nucleic acid strand or the second blocking nucleic acid strand. For example, the third blocking nucleic acid strand might comprise cleavable linkers. In particular, there might be a plurality of the third blocking nucleic acid strands, wherein the third blocking nucleic acid strands of the plurality of blocking nucleic acid strands are linked to each other by cleavable linkers, in particular in order to form a cleavable linear chain of the blocking nucleic acid strands. The linked third blocking nucleic acid strands of the third plurality may form a third cleavable linear chain of blocking nucleic acid strand. The cleavable linkers may be selectively cleavable by applying a cleavage agent.

In particular, providing the label with the third nucleic acid strand requires the specific hybridisation of three nucleic acid strands to each in order to generate the label. In particular in case the labelling moieties are attached to the third nucleic acid strand, this enables detecting the label only if the three nucleic acid strands are hybridised to each other and the triplex structure has formed. Additionally, the triplex structure enables providing a robust and rigid structure, to which the labelling moieties may be attached.

The third nucleic acid strand may have a third nucleotide sequence, in particular. The third nucleotide sequence may be at least partially complementary to the hybridised first nucleotide sequence and second nucleotide sequence, in particular based on Hoogsteen base-pairing, forming the triplex structure. Thus, the third nucleotide sequence can hybridise to the duplex of the first nucleotide sequence and the second nucleotide sequence, in particular to a major groove of the duplex. This enables a stable triplex.

In an embodiment, each labelling moiety is equally spaced from any adjacent labelling moiety. This enables generating an optically detectable signal that is proportional to the distance between the first label part and the second label part. When the label comprises a plurality of labelling moieties, each first labelling moiety is substantially equally spaced from any adjacent first labelling moiety, and/or each second labelling moiety is substantially equally spaced from any adjacent second labelling moiety. Preferably, the essentially equal spacing may be in a range from 0.33 nm to 33 nm.

In a further aspect a marker for analysing a biological sample with a plurality of target analytes is provided. The marker comprises a label, in particular as described above, comprising a first label part and a second label part. The marker further comprises a first marker part comprising a first affinity reagent and the first label part, and a second marker part comprising a second affinity reagent and the second label part. The first affinity reagent and the second affinity reagent are each configured to bind specifically to one of the target analytes of the biological sample. Optionally, the marker may comprise a third marker part comprising the third label part with the third nucleic acid strand.

The marker enables efficiently determining the distance or proximity between two target analytes. The marker also enables precisely determining the presence and/or location of the two target analytes or of a single target analyte in a biological sample. Additionally, the at least one blocking nucleic acid strand enables handling of the first and second marker parts without the risk of undesirable uncontrolled hybridisation between the first and second nucleic acid strands, for example when preparing to analyse a biological sample with the marker.

The biological sample may be a cellular sample, such as a tissue section, for example. Further examples include biopsy samples such as liquid biopsy or a tissue biopsy. The target analyte may be a protein or a nucleic acid of the biological sample, for example. The affinity reagents of the marker may be antibodies, antibody fragments, amino acid- or nucleic acid-based aptamers, or linear nucleic acids. This enables detecting a large variety of target analytes. In particular, the nucleic acid strand of the first and second label part is attached to the respective affinity reagent, preferably covalently attached.

The affinity reagents of the marker may bind to a single target analyte or to separate target analytes. In the first case, the first affinity reagent may be configured to bind specifically to a first area or epitope of the single target analyte and the second affinity reagent may be configured to bind specifically to a second area or epitope of the single target analyte. By requiring two marker parts to bind to the single target analyte, the presence and/or location of the single target analyte may be precisely determined, in particular with an increased specificity. In the second case, the first affinity reagent may be configured to bind specifically to a first target analyte and the second affinity reagent may be configured to bind specifically to a second target analyte. This enables determining the distance or proximity between the first target analyte and the second target analyte.

In particular when the marker comprises the third marker part and the labelling moieties are attached to the third nucleic acid strand, only the presence of the three marker parts and the formation of the triplex results in the marker being detectable. This enables detecting the single target analyte with an increased specificity. Similarly, in case of determining the proximity of the first and second target analyte, the third marker part enables detection with an increased specificity.

In another aspect, a method for analysing a biological sample is provided. The method comprises introducing at least one marker, in particular as described herein, into the biological sample. Optionally, a first optical readout of the biological sample with the marker may be generated, in particular prior to the step of removing the blocking nucleic acid strand from the marker. In a further step, the at least one blocking nucleic acid strand is removed from the marker. In a subsequent step a further optical readout of the biological sample with the marker is generated. In a subsequent optional step, the first optical readout and the further optical readout are compared, in particular in order to determine a change in optical properties of the marker. Specifically, a fold change, in particular of a fluorescence emission of the marker, between the first and further optical readouts may be calculated.

The optical readouts may be generated by means of a microscope, such as a fluorescent microscope, for example. The optical readout enables determining the presence and/or location of a target analyte or a proximity of two target analytes.

The step of introducing the at least one marker may optionally include waiting for a set amount of time to allow binding of the at least one marker to a respective target analyte or analytes of the biological sample. After introducing the at least one marker, any marker not bound to the target analyte may optionally be removed from the biological sample, for example by washing. When introducing the at least one marker, the marker comprises the blocking nucleic acid strand, preferably the blocking nucleic acid strand is hybridised to the respective first or second nucleic acid strand of the label of the marker. The step of introducing the at least one marker may include adding individual parts of the at least one marker to the sample separately, in particular separately over time. In this case, the marker is assembled in situ of the sample. Alternatively, the individual parts of the marker may be added to the sample together or at the same time.

After the step of introducing the at least one marker, preferably after the binding of the at least one marker to a respective target analyte of the biological sample and in particular after any marker not bound to the target analyte has been removed from the biological sample, for example by washing, the first optical readout of the biological sample with the marker may be generated. This enables optically reading out the single marker or label parts (e.g. the first and the second marker or label parts) before the next method step - the removal of the blocking nucleic acid strand from the marker - is performed. While it may not always be required, it is generally advisable to perform at least two optical readouts of the biological sample: one wherein the first and/or second label part are still hybridised to the blocking nucleic acid strands and one after the removal of the blocking strands. In this way a fold change or ratio in a fluorescence emission may be calculated.

The step of removing the at least one blocking nucleic acid strand may include applying a cleavage agent in order to cleave the cleavable linkers of the at least one marker.

This enables removing the blocking nucleic acid strands due to a decrease in their melting temperature. In particular, the individual blocking nucleic acid strands may be displaced by the first/second nucleic acid strands. Additionally, the step of removing the at least one blocking nucleic acid strand may include washing the sample to remove excess blocking strands. Additionally, the step may include increasing the sample temperature to a temperature in a range of 37°C to 42°C.

The method for analysing the biological sample and the marker have the same advantages as the label. Further, the method and marker may be supplemented with the features of the label described in this document, in particular, the features of the dependent claims of the label.

### Short Description of the Figures

Hereinafter, specific embodiments are described referring to the drawings, wherein:
- Figure 1: is a schematic view of a marker comprising a label for analysing a biological sample,
- Figure 2: is a schematic view of the marker according to Fig. 1,
- Figure 3: is a schematic view of the marker according to Fig. 1,
- Figure 4: is a schematic view of the marker according to Fig. 1,
- Figure 5: is a schematic view of a marker according to a second embodiment,
- Figure 6: is a schematic view of a marker according to a third embodiment,
- Figure 7: is a schematic view of a marker according to a fourth embodiment,
- Figure 8: is a schematic view of the marker according to Fig. 7,
- Figure 9: is a flow chart of a method for analysing a biological sample,
- Figure 10: is a schematic view of a use of a marker for flow cytometry,
- Figure 11: is a schematic view showing the use of proximity hybridization assays (PHA) for assessing genetic modifications, and
- Figure 12: is a schematic view showing the use of proximity hybridization assays (PHA) for gene and cell therapy quality control and monitoring.

### Detailed Description

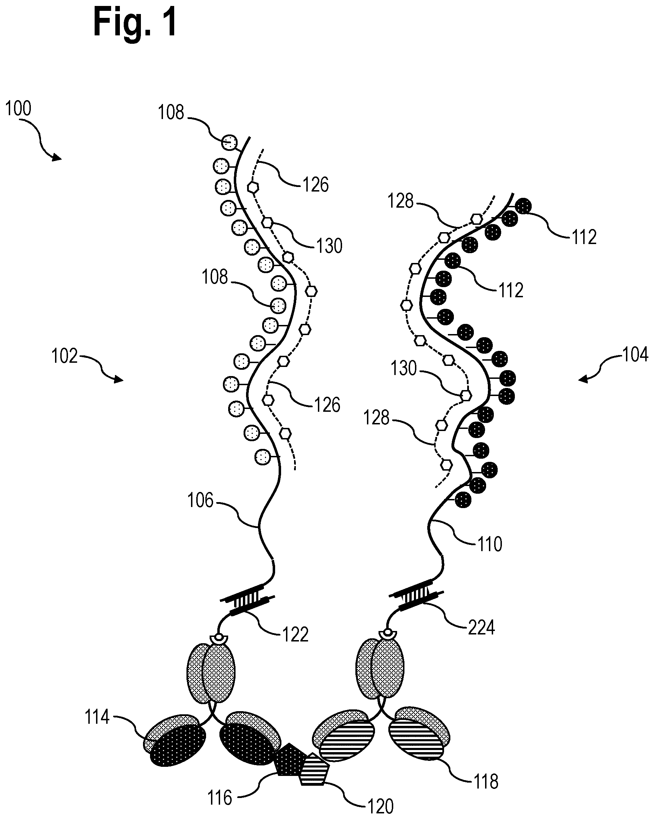

Figure 1 is a schematic view of a marker 100 for analysing a biological sample (not shown). The marker 100 comprises a first marker part 102 and a second marker part 104. The first marker part 102 may comprise a first label part with a first nucleic acid strand 106, and a plurality of first labelling moieties 108. The first labelling moieties 108 may be (covalently) attached to the first nucleic acid strand 106.

The second marker part 104 comprises a second label part with a second nucleic acid strand 110, and a plurality of second labelling moieties 112 attached to the second nucleic acid strand 110. The first nucleic acid strand 106 and the second nucleic acid strand 110 may be polynucleotides, for example.

The first nucleic acid strand 106 and the second nucleic acid strand 110 are at least partially complementary to each other. This enables hybridisation of the first nucleic acid strand 106 and the second nucleic acid strand 110, in particular to form a duplex based on Watson-Crick base pairing.

The first marker part 102 further comprises a first affinity reagent 114 configured to specifically bind to a first target analyte 116. Similarly, the second marker part 104 further comprises a second affinity reagent 118 configured to specifically bind to a second target analyte 120.

The first label part is attached to the first affinity reagent 114 via a barcode oligonucleotide 122 of the first affinity reagent 114. Similarly, the second label part is attached to the second affinity reagent 118 via a barcode oligonucleotide 124. Each barcode oligonucleotide 122, 124, in particular its sequence of nucleotides, is preferably specific to the respective affinity reagent 114, 118 and/or the respective label part, in particular to the first and second nucleic acid strand 106, 110, respectively. In particular, the first and second affinity reagent 114, 118 are antibodies.

The first marker part 102 is bound to the first target analyte 116 via the first affinity reagent 114. Similarly, the second marker part 104 is bound to the second target analyte 120 via the second affinity reagent 118.

The marker 100 further comprises a first plurality of blocking nucleic acid strands 126 and/or a second plurality of blocking nucleic acid strands 128. The blocking nucleic acid strands 126, 128 are configured to either hybridise to the first nucleic acid strand 106 or the second nucleic acid strand 110. In Fig. 1 the first blocking nucleic acid strands 126 are shown to be hybridised to the first nucleic acid strand 106 and the second blocking nucleic acid strands 128 are shown to be hybridised to the second nucleic acid strand 110.

The first plurality of blocking nucleic acid strands 126 are linked to each other by means of cleavable linkers 130. This forms a linear chain of the blocking nucleic acid strands 126 that is complementary to the first nucleic acid strand 106. The cleavable linkers 130 may form covalent links between adjacent blocking nucleic acid strands 126, 128

The cleavable linkers 130 are configured to be specifically cleaved by a cleavage agent. For example, the cleavable linkers 130 may be a photocleavable linker configured to be cleaved by UV light. An example of such a photocleavable linker is BMN linker (Biomers). Preferably, the cleavable linker is not or does not comprise nucleotides. Alternatively, the cleavable linker 130 may be a chemically or enzymatically cleavable linker.

Similarly to the blocking nucleic acid strands 126, the second plurality of blocking nucleic acid strands 128 are linked to each other by means of the cleavable linkers 130.

The presence of the linked blocking nucleic acid strands 126, 128 and their hybridisation to the respective nucleic acid strand 106, 110 means that the first nucleic acid strand 106 and the second nucleic acid strand 110 are prevented from directly hybridising to each other. Thus, when handling the marker parts 102, 104, for example, when preparing to analyse a biological sample, the marker parts 102, 104 do not hybridise to each other due to the linked blocking nucleic acid strands 126, 128.

In an alternative embodiment, the marker 100 may only comprise either one of the first or second plurality of blocking nucleic acid strands 126, 128. This single plurality of blocking nucleic acid strand 126, 128 may nevertheless achieve the abovementioned effect of hindering the hybridisation of the first nucleic acid strand 106 with the second nucleic acid strand 108.

In the exemplary embodiment of Fig. 1, the target analytes 116, 120 are in contact, for example, due to an affinity interaction between the target analytes 116, 120. This results in the marker parts 102, 104 to be in proximity once they are bound to the target analytes 116, 120. As described above, despite their proximity, the first nucleic acid strand 106 and the second nucleic acid strand 110 do not hybridise to each other due to the blocking nucleic acid strands 126, 128. This enables easy handling of the marker 100, in particular its marker parts 102, 104 during an analysis of a biological sample containing the target analytes 116, 120. For example, in case the marker parts 102, 104 are not yet bound to the target analytes 116, 120 when introducing the marker 100 into a biological sample, the blocking nucleic acid strands 126, 128 prevent the undesired uncontrolled hybridisation of the first nucleic acid strand 106 and the second nucleic acid strand 110.

The first labelling moieties 108 and the second labelling moieties 112 may be fluoro-phores that act as FRET donors or FRET acceptors, respectively. Thus, each first labelling moiety 108 may form a FRET pair with one of the second labelling moieties 112. Since the first nucleic acid strand 106 and the second nucleic acid strand 110 are not hybridised in the state shown in Fig. 1, the first nucleic acid strand 106 and the second nucleic acid strand 110 are not in close proximity to each other. In particular, the first labelling moieties 108 and the second labelling moieties 112 are not within a distance that enables a FRET to occur. Such a distance is frequently in the range between 1 and 10 nm. Thus, the first labelling moieties 108 and the second labelling moieties 112 may be optically detectable as (single) ordinary fluorescent dyes but not according to the FRET characteristic.

In particular, Fig. 1 shows a step of a method for analysing a biological sample according to which the marker 100 may be introduced into a biological sample to be analysed. The blocking nucleic acid strands 126, 128 prevent undesired hybridisation of the first and second nucleic acid strands 106, 110 before the marker 100 is introduced into the biological sample and the affinity reagents 114, 118 have bound to their respective target analytes 116, 120.

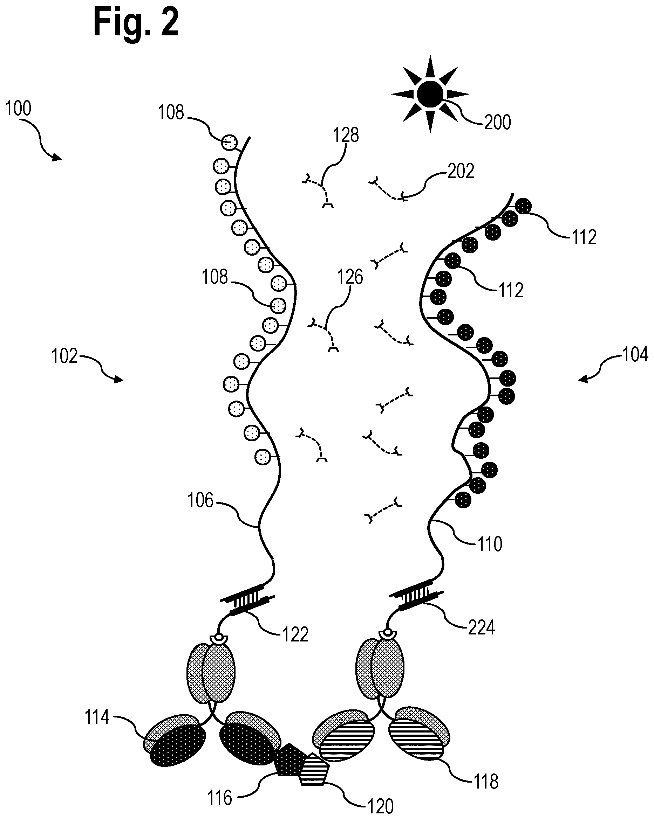

Figure 2 is a schematic view of the marker 100 in the presence of a cleavage agent 200. In particular, the cleavage agent 200 is UV light, with which the marker 100, in particular the cleavable linkers 130 are illuminated. The cleavage agent 200 can (specifically) cleave the cleavable linkers 130. This results in the individual blocking nucleic acid strands 126, 128 being separated and only residuals 202 of the linkers 130 remaining attached to the blocking nucleic acid strands 126, 128. In particular, the cleavage agent 200 may be applied to a biological sample to be analysed as part of a method for analysing the biological sample.

Generally, the hybridisation of longer nucleic acids with each other is thermodynamically more stable than the hybridisation of shorter nucleic acids. Thus, the hybridisation of the linked blocking nucleic acid strands 126, 128 to either first or second nucleic acid strands 106, 110 is thermodynamically more stable than the hybridisation of the respective cleaved, single blocking nucleic acid strands 126, 128 to the first or second nucleic acid strands 106, 110.

Preferably, the individual blocking nucleic acid strands 126, 128 each have a number of nucleotides in the range of 5 to 15 nucleotides. A number of the blocking nucleic acid strands 126, 128 may be linked to result in respective linear chains of the blocking nucleic acid strands 126, 128. In particular, 5 to 30 blocking nucleic acid strands 126, 128 may be linked to each other by linkers 130.

Thus, by cleaving the blocking nucleic acid strands 126, 128 off each other, the duplex formed between the blocking nucleic acid strands 126, 128 and the respective nucleic acid strand 106, 110 can be destabilised. This may result in the blocking nucleic acid strands 126, 128 dissociating from the nucleic acid strands 106, 110 and/or the duplex formation between the nucleic acid strands 106, 110 displacing the blocking nucleic acid strands 126, 128.

In addition, to further destabilise the duplex formed between the individual blocking nucleic acid strands 126, 128 and the respective nucleic acid strand 106, 110, the temperature the marker is at may be increased, in particular near or above an average melting temperature of the duplex of the individual blocking nucleic acid strands 126, 128 and the respective nucleic acid strands 106, 110.

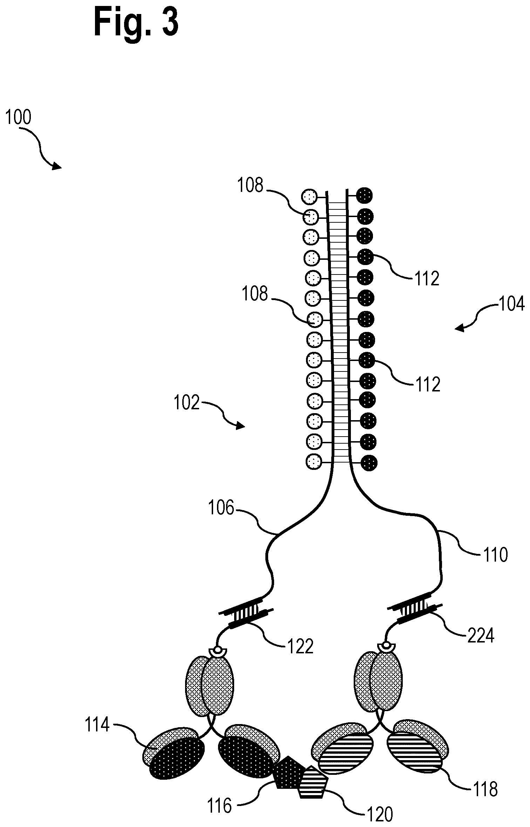

Figure 3 is a schematic view of the marker 100 with the blocking nucleic acid strands 126, 128 removed, for example, due to the previous addition of the cleavage agent 200 as described for Fig. 2. Without the blocking nucleic acid strands 126, 128 present, the first and second nucleic acid strands 106, 110 may freely hybridise to each other to form the duplex. In Fig. 3 the first and second nucleic acid strands 106, 110 are shown to be hybridised to each other.

The hybridisation of the first nucleic acid strand 106 and the second nucleic acid strands 110 brings the first labelling moieties 108 and the second labelling moieties 112 into proximity such that a FRET may occur between the first labelling moieties 108 and the second labelling moieties 112. Thus, the proximity of the target analytes 116, 120 may be determined by the occurrence of FRET between the first labelling moieties 108 and the second labelling moieties 112, for example, by means of an optical readout with a fluorescence microscope. In Fig. 3, a FRET can occur between all the first labelling moieties 108 and the second labelling moieties 112.

In a method for analysing a biological sample, the marker 100, in particular the marker parts 102, 104, may initially be introduced into the biological sample in the presence of the blocking nucleic acid strands 126, 128, as shown in Fig. 1. After waiting for an appropriate amount of time for the marker parts 102, 104 to bind, any marker parts 102, 104 that remain unbound to a respective target analyte 116, 118 may be removed, for example by washing the biological sample.

Subsequently, the blocking nucleic acid strands 126, 128 may be cleaved as described for Fig. 2, which results in their dissociation. The blocking nucleic acid strands 126, 128 may further be removed from the sample, for example by washing the sample. Fig. 3 shows the marker 100 after removal of the blocking nucleic acid strands 126, 128.

This in turn enables hybridisation of the first nucleic acid strand 106 with the second nucleic acid strands 110. Subsequently, an optical readout may be generated of the biological sample with the marker 100. The optical readout may be an image, for example, generated by means of a fluorescence microscope.

The optical readout enables determining whether or not the target analytes 116, 120 are in close proximity to each other. In particular, the optical readout can be used to determine whether or not a FRET occurs between the first labelling moieties 108 and the second labelling moieties 112, by observing a respective change in their optical properties due to the FRET.

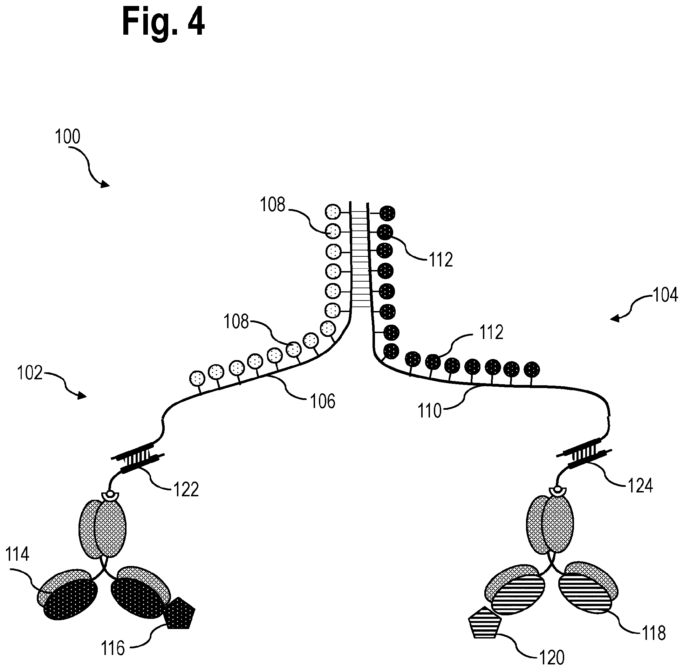

Figure 4 is a schematic view of the marker 100 with the target analytes 116, 120 at a greater distance from each other compared to the Figs. 1 to 3. Similarly to Fig. 3, the blocking nucleic acid strands 126, 128 are not present and the first nucleic acid strand 106 and the second nucleic acid strand 110 can hybridise to each other. However, the greater distance between the target analytes 116, 120 results in the first nucleic acid strand 106 and the second nucleic acid strand 110 to only partially hybridise, in particular to hybridise to a lesser extent compared to the state shown in Fig. 3, in which the target analytes 116, 120 are in contact with each other.

The partially hybridised first nucleic acid strand 106 and second nucleic acid strand 110 results in some of the first labelling moieties 108 and the second labelling moieties 112 to be at a greater distance from each other. In particular, these first labelling moieties 108 and second labelling moieties 112 may be at a distance from each other, at which no FRET occurs between them. Thus, the overall observable FRET efficiency is reduced compared to the state shown in Fig. 3. This reduced FRET efficiency can be observed when generating an optical readout of the biological sample with the marker 100. Thus, the first labelling moieties 108 and the second labelling moieties 112 which do not form a FRET pair - e.g. the labelling moieties 108, 112 between the barcode oligonucleotides 122, 124 and the hybridised part of the first nucleic acid strand 106 and the second nucleic acid strand 110 - might be optically detectable as (single) ordinary fluorescent dyes according to their (spectral) emission characteristic of the fluorescent dyes, whereas the first labelling moieties 108 and the second labelling moieties 112 which do form a FRETR pair - e.g. the labelling moieties 108, 112 of the hybridised part of the first nucleic acid strand 106 and the second nucleic acid strand 110 - can be detected according to the FRET characteristic of the FRET pair and therefore according to a different spectral characteristic of the FRET pair compared to the spectral characteristic of the single fluorescent dyes. The ratio between the two different spectral characteristics (the spectral characteristics of the FRET pair vs. the individual fluorescent dyes) can be used as a quantitative measure of the respective proximity of the affinity reagents 114, 118 and therefore of the target analytes 116, 120.

In summary and with particular reference to Figs. 3 and 4, it can be seen that the distance between the target analytes 116, 120 has a direct effect on the degree of hybridisation between the first nucleic acid strand 106 and second nucleic acid strand 110 and therefore on the overall FRET efficiency between the first labelling moieties 108 and second labelling moieties 112. The FRET efficiency between the first labelling moieties 108 and second labelling moieties 112 is therefore proportional to the distance between the target analytes 116, 120 and can be used to determine the distance or proximity between the target analytes 116, 120.

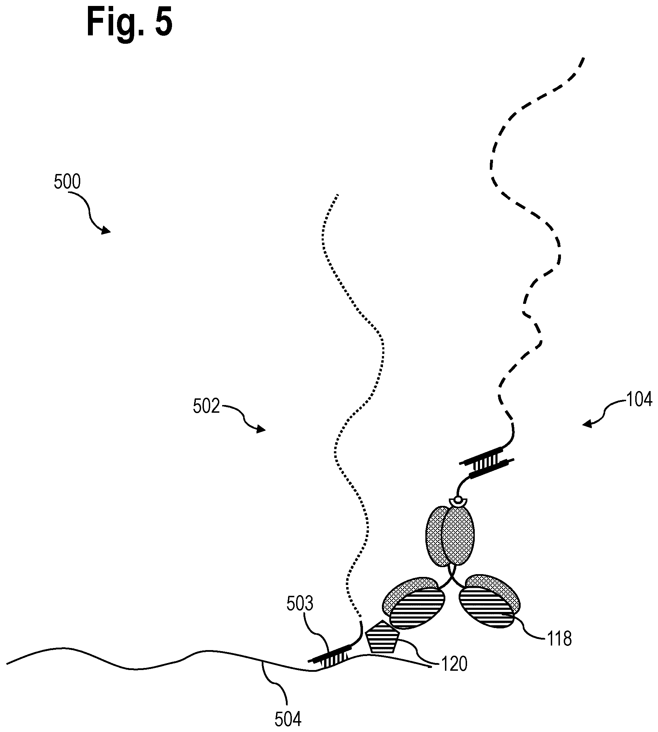

Figure 5 is a schematic view of a marker 500. The marker 500 comprises a first marker part 502 with an affinity reagent 503 that is a linear nucleic acid. The marker 500 further comprises the second marker part 104 described above. For simplicity no labelling moieties are shown in Fig. 5 for the marker 500. The marker 500 may further comprise blocking nucleic acid strands 126, 128, as described above. In Fig. 5, the marker 500 is shown without the blocking nucleic acid strands 126, 128.

The first marker part 502 binds specifically to a nucleic acid target analyte 504. The second marker part 104 binds specifically to the second target analyte 120, which may be a protein. The target analytes 504, 120 may interact with each other or bind to each other. This interaction or proximity between the target analytes 504, 120 may be determined by the marker 500 in the same manner as described for the marker 100. In particular, the proximity of respective labelling moieties (not shown) of the marker parts 502, 104 may be determined by FRET occurring between the labelling moieties as a measure of the distance between the target analytes 504, 120.

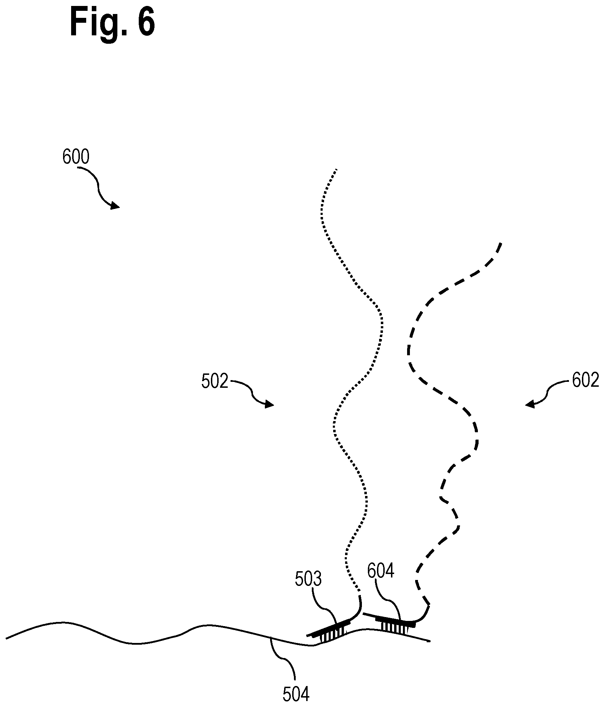

Figure 6 is a schematic view of a marker 600. The marker 600 comprises the first marker part 502 and a second marker part 602 with first and second affinity reagents 503, 604 that are linear nucleic acids, respectively. For simplicity no labelling moieties are shown in Fig. 6 for the marker 600. The marker 600 may further comprise blocking nucleic acid strands 126, 128, as described above. In Fig. 6, the marker 600 is shown without the blocking nucleic acid strands 126, 128.

Both affinity reagents 503, 604 are configured to specifically bind to a particular target sequence. The nucleic acid target analyte 504 comprises these target sequences. By means of the marker 600 the proximity between the target sequences along the target analyte 504 may be determined or the presence of the target analyte 504 may be determined with high confidence due to the use of two affinity reagents that need to bind to the target analyte 504 simultaneously. The marker 600 may be detected as described for the markers 100, 600 above. In particular, the proximity of respective labelling moieties (not shown) of the marker parts 502, 602 may be determined by FRET occurring between respective labelling moieties (not shown) of the marker parts 502, 602 as a measure of the distance between the target sequence or as a measure of the presence of the target analyte 504 in the biological sample.

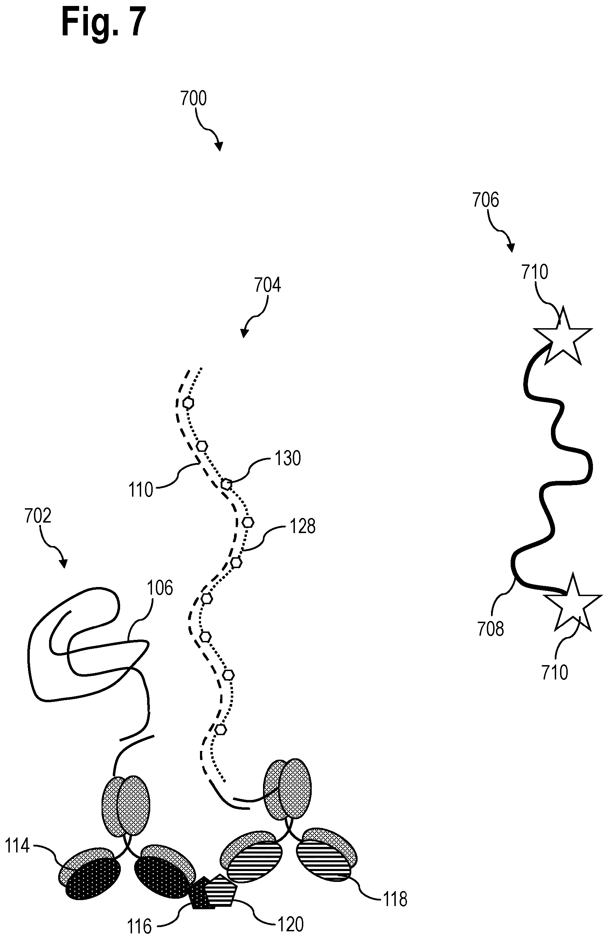

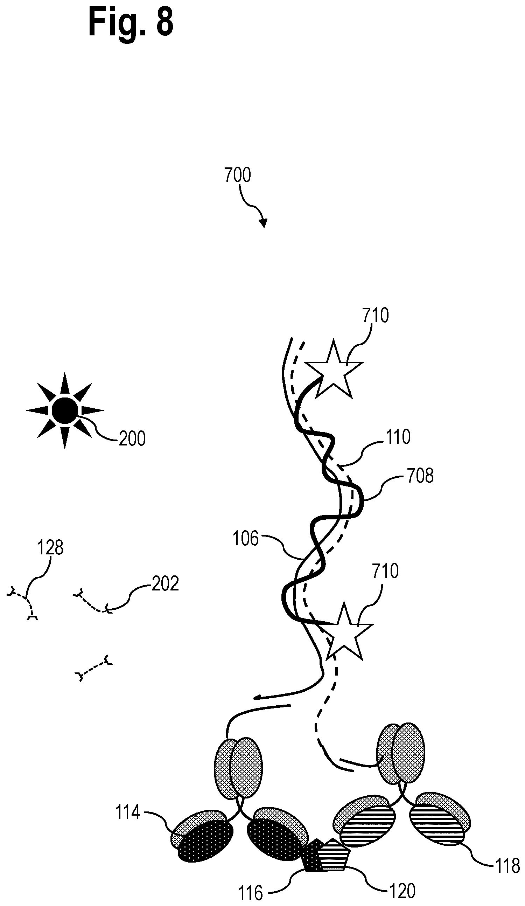

Figures 7 and 8 are schematic views of a marker 700 comprising a first marker part 702, a second marker part 704 and a third marker part 706. The first marker part 702 comprises the first nucleic acid strand 106 and the second marker part 704 comprises the second nucleic acid strand 110. The third marker part 706 comprises a third nucleic acid strand 708 configured to form a triplex with the first and second nucleic acid strands 106, 110, in particular based on Hoogsteen base-pairing. Two labelling moieties 710 are attached to the third nucleic acid strand 708.

In Figure 7, the marker 700 is shown with the second plurality of blocking nucleic acid strands 128, linked to each other by linkers 130, hybridised to the second nucleic acid strand 110. This results in the marker parts 702, 704, 706 being prevented from hybridising to each other. In particular, neither the duplex between the first and second nucleic acid strands 106, 110 nor the triplex between the third nucleic acid strand 708 and the first and second nucleic acid strands 106, 110 can be formed.

In Figure 8, the marker 700 is shown with the linkers 130 cleaved by applying the cleavage agent 200 and the blocking nucleic acid strands 128 separated from each other. This enables formation of the triplex between the first, second and third nucleic acid strands 106, 110, 708. In particular, this associates the labelling moieties 710 with the affinity reagents 114, 118 and therefore with the target analytes 116, 120. This enables determining the presence and/or location of the target analytes 116, 120 in an optical readout of the sample. Simultaneously, the target analytes 116, 120 are required to be in close proximity in order to enable formation of the triplex. The labelling moieties 710 may either have the same optical properties or have different optical properties.

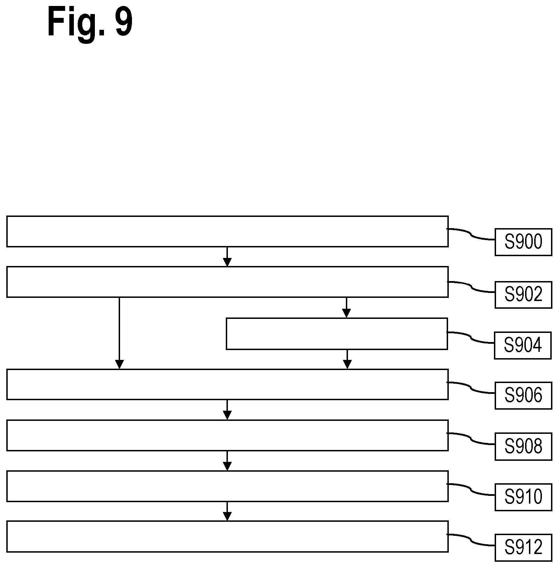

Figure 9 is a schematic view of a flow chart of a method for analysing a biological sample. The method starts in step S900.

In step S902 at least one marker 100, 500, 600, 700 as described herein is introduced into the biological sample. In particular, a plurality of markers may be introduced into the sample, each marker comprising affinity reagents that are configured to specifically bind to different target analytes. In this step, the linked blocking nucleic acid strands are bound to respective nucleic acid strands of the marker parts. This enables introducing the marker parts into the biological sample without the risk of the marker parts hybridising to each other before they bind to their respective target analytes.

Additionally and subsequently, the step S902 may comprise removing markers parts that are unbound to any target analytes, for example by washing the sample.

In an optional step S904, a first optical readout is generated of the sample with the markers, for example by means of a microscope. This is of particular relevance when the marker 100 was introduced into the sample in step S902. The first optical readout enables determining the fluorescence of the labelling moieties 108, 112 when the marker parts 102, 104 are prevented to form a duplex due to the presence of the linked blocking nucleic acid strands 126, 128. Thus, the labelling moieties 108, 112 have their native optical properties, without a FRET occurring.

In a step S906, the blocking nucleic acid strands are removed. This includes cleaving the cleavable linkers 130 between individual blocking nucleic acid strands, for example by applying a cleavage agent such as UV light. The cleaving causes the blocking nucleic acid strands to be individualised and the duplex between the blocking nucleic acid strands and the respective nucleic acid strands of the marker parts to be destabilised.

The step S906 may optionally include changing a condition at which the sample is kept in order to further destabilise the duplex between the blocking nucleic acid strands and the respective nucleic acid strands of the marker parts. The changed condition may be a physical or chemical condition, for example, a temperature of the sample or a buffer/salt concentration at which the sample is kept. In particular, the temperature of the sample may be increased to a temperature in a range of 37°C to 42 °C.

As a further option, the cleaved blocking nucleic acid strands may be removed from the sample, for example by washing the sample.

The removal of the blocking nucleic acid strands enables formation of the duplex between the nucleic acid strands of the marker parts.

In step S908 a further optical readout of the sample with the marker is generated, for example by means of a microscope. For example in case of the marker 100 and the target analytes 116, 120 being in close proximity, the marker parts 102, 104, in particular their respective nucleic acid strands 106, 110 are now hybridised to each other. This results in the labelling moieties 106, 110 forming a FRET pairs, which may be optically detected in the optical readout.

In step S910 the presence and/or location of the target analytes can be determined in the sample based on an optical signal of the labelling moieties being present in at least the further optical readout. For example in case of the marker 700, the optical signal of the labelling moieties 710 can be determined in the further optical readout and based on that the presence and/or location of the target analytes 116, 120 can be determined. Additionally, it can be determined that the target analytes 116, 120 are in proximity to each other.

Optionally and in case of the marker 100, the first optical readout can be compared to the further optical readout and a difference in the optical properties of the labelling moieties 108, 112 may be used to determine the presence, location and/or proximity of the target analytes 116, 120.

The method ends in step S912.

The method steps S902 to S910 may be iteratively repeated, with a different set of markers in each iteration. In this case, the label parts of the markers may preferably be removed after each iteration in order to remove the labelling moieties of the respective markers.

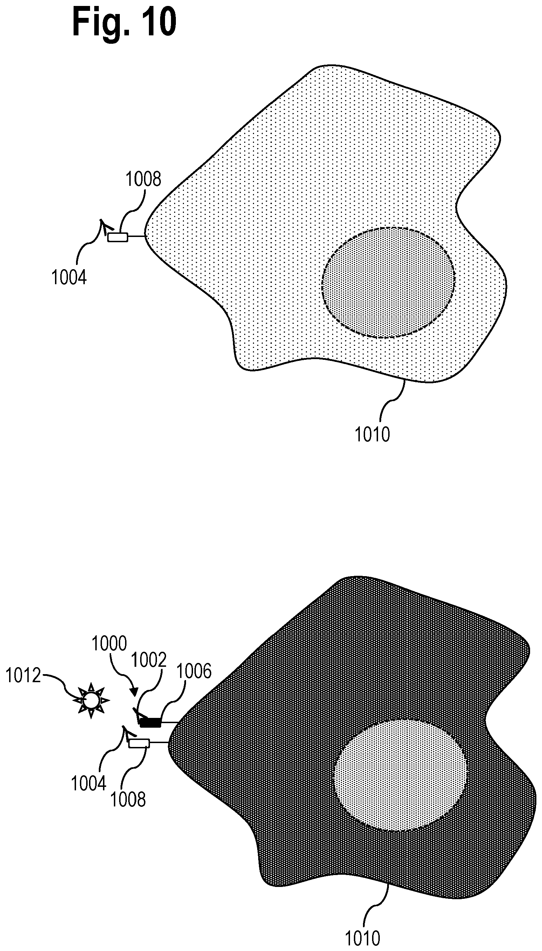

Figure 10 is a schematic view of a use of a marker 1000 for flow cytometry. The marker 1000 comprises marker parts 1002, 1004 that bind to respective targets 1006, 1008 presented or expressed on a cell's surface 1010. In case both targets 1006, 1008 are present on the cell's surface 1010, both marker parts 1002, 1004 bind and the marker 1000 generates an optically detectable signal 1012. In case only one of the targets 1008 is present on the cell's surface 1010, only the marker part 1004 binds and no optically detectable signal is generated. Thus, cells may be contacted with the marker 1000 prior to flow cytometry in order to distinguish between cells that have one or both targets 1006, 1008.

The marker 1000 may further be used to determine whether two cells, each having either the target 1006 or the target 1008, are in close proximity, in particular whether the targets 1006, 1008 are in proximity on the surfaces of the cells.

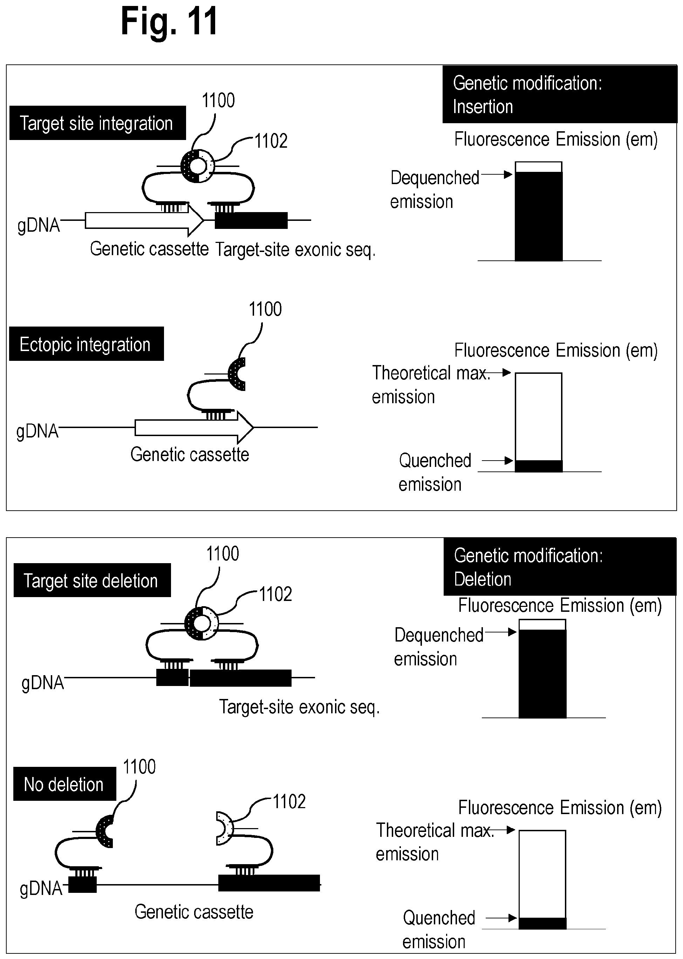

Figure 11 is a schematic view of a PHA assay that is used for assessing the integration of genetic cassettes that are used in gene and cell therapy. These assays are based on using oligonucleotides as affinity reagents and single molecule fluorescent in situ hybridization (smFISH). In Figure 11 the first and second label parts are schematically shown as half donut-shapes. Respective markers may comprise blocking nucleic acid strands in order to prevent the first and second label parts to hybridise to each other.

The top row of the top panel of Fig. 11 shows the target site integration of the genetic cassette (white arrow) next to an exon (black rectangle) the genomic DNA is otherwise depicted as a black line. The bottom row of the top panel of Fig. 11 shows a random or ectopic integration. PHA assays allow the faithful separation of the distinct classes of integrations as only the target site integrations are detected with both label parts 1100, 1102 of the marker, whereas in a random integration only the first or second label part 1100, 1102 is present. Following to the removal of the blocking strands the target site integration is detectable as a fluorescent spot, which is brighter than the one of an ectopic integration when dequenching-based PHA assays are used and/or which has a higher FRET efficiency or different fluorescent lifetime, when FRET-based PHA assays are used.

The bottom panel of Fig. 11 shows a schematic view of a PHA assay that is used for assessing the deletion of genomic sequences. A part of the genome may for example be deleted in a cell line to correct a genetic defect. This can be achieved by for example using the CRISPR/Cas9 or similar systems as well as by leveraging homologous recombination. If patient-derived cells for example are modified to delete a certain part of the genome it may be desirable to assess the frequency which with the appropriate deletion occurs. This may be achieved by using the PHA assay schematically shown in the bottom panel. Due to the deletion of the target site both probes of the first and second marker part are brought into proximity, which can be readout out as a corresponding smFISH spot, will be brighter than the one of a site that has no deletion when dequenching-based PHA assays are used and/or which has a higher FRET efficiency or different fluorescent lifetime, when FRET-based PHA assays are used.

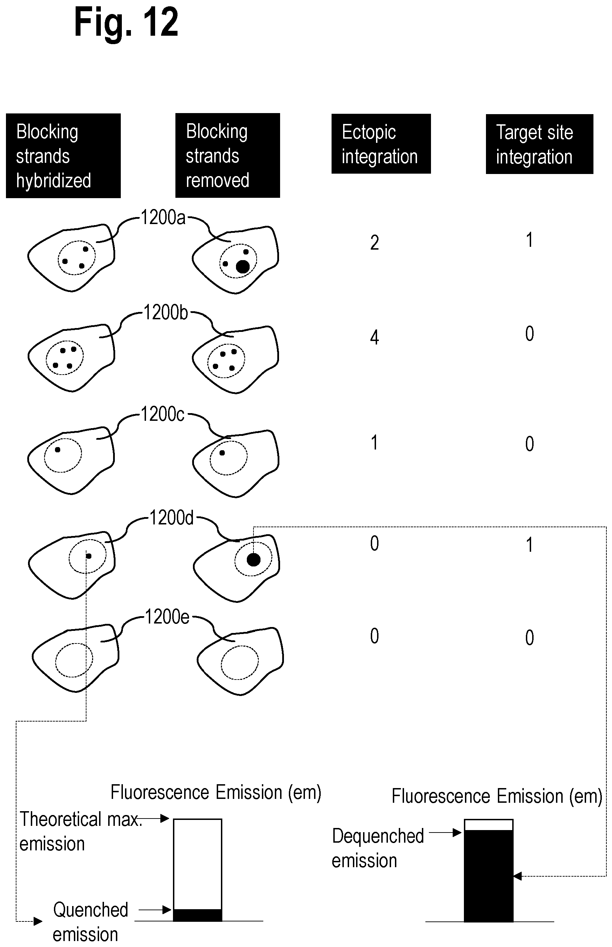

Figure 12 is a schematic view of a hypothetical readout of the assay described in Figure 11. A plurality of cells 1200a, 1200b, 1200c, 1200d, 1200e is analysed, which may for example be derived from a blood sample to monitor the evolution of a cell therapy product in a patient. Such a monitoring may be performed to assess how distinct clones in a cell therapy product evolve in a patient during the course of a cell therapy. Such clones may comprise no genetic modification (e.g. no insertion/integration or deletion), they may comprise only the desired target-site modification, they may comprise only ectopic integrations and/or deletions and they may comprise a mix of target-site and ectopic modification. Cells are stained and readout before and after removal of the blocking strands, spots are segmented and the before and after images are registered to identify the spots that change following to the removal of the blocking strands indicating a paired detection event.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of Reference Signs

- 100, 500, 600, 700, 1000: Marker
- 102, 502, 702, 1002, 1100: First marker part
- 104, 602, 704, 1004, 1102: Second marker part
- 106: First nucleic acid strand
- 108, 710: First labelling moiety
- 110: Second nucleic acid strand
- 112: Second labelling moiety
- 114, 503: First affinity reagent
- 116, 504, 1006: First target analyte
- 118, 604: Second affinity reagent
- 120, 1008: Second target analyte
- 122, 124, 224: Barcode oligonucleotide
- 126: Blocking nucleic acid strand of first plurality
- 128: Blocking nucleic acid strand of second plurality
- 130: Cleavable linker
- 200: Cleavage agent
- 202: Residual of linker
- 706: Third marker part
- 708: Third nucleic acid strand
- 1010: Cell surface
- 1012: Optical signal
- 1200a, 1200b, 1200c, 1200d, 1200e: Cell of a plurality of cells

## Claims

1. A label for analysing a biological sample comprising:
a first label part comprising a first nucleic acid strand (106), and
a second label part comprising a second nucleic acid strand (110), and
wherein the first nucleic acid strand (106) and the second nucleic acid strand (110) are configured to form a duplex,
wherein the label further comprises at least one first labelling moiety (108, 710) and at least one second labelling moiety (112, 710), and
wherein the label further comprises at least one blocking nucleic acid strand (126, 128) at least partially complementary to one of the first nucleic acid strand (106) and the second nucleic acid strand (110).

2. The label according to claim 1, comprising a plurality of the blocking nucleic acid strands (126, 128), wherein the blocking nucleic acid strands (126, 128) of the plurality of blocking nucleic acid strands (126, 128) are linked to each other by cleavable linkers (130).

3. The label according to claim 2, wherein the cleavable linkers (130) are photocleavable linkers, chemically cleavable linkers, or enzymatically cleavable linkers.

4. The label according to one of the preceding claims 2 to 3, wherein all the blocking nucleic acid strands (126, 128) of the plurality of blocking nucleic acid strands are complementary to respective nucleotide sequences of either the first nucleic acid strand (106) or the second nucleic acid strand (110).

5. The label according to one of the preceding claims 2 to 4, wherein adjacently linked blocking nucleic acid strands (126, 128) of the plurality of blocking nucleic acid strands are complementary to respective adjacent nucleotide sequences of the first nucleic acid strand (106) or the second nucleic acid strand (110).

6. The label according to one of the preceding claims 2 to 5, wherein each of the at least one blocking nucleic acid strands (126, 128) comprises a number of nucleotides in a range of 5 to 15 nucleotides, preferably, 5 to 12 nucleotides, more preferably, 6 to 9 nucleotides.

7. The label according to one of the preceding claims, wherein the first nucleic acid strand (106) and/or the second nucleic acid strand (110) comprise a number of nucleotides in a range of 50 to 150 nucleotides.

8. The label according to one of the preceding claims, comprising a second plurality of blocking nucleic acid strands (128), wherein the blocking nucleic acid strands (128) of the second plurality of blocking nucleic acid strands are linked to each other by cleavable linkers (130), and wherein the blocking nucleic acid strands (128) of the second plurality of blocking nucleic acid strands are complementary to respective nucleotide sequences of the second nucleic acid strand (110).

9. The label according to one of the preceding claims, wherein the at least one blocking nucleic acid strand (126, 128) is complementary to a part of the first nucleic acid strand (106) or a part of the second nucleic acid strand (110) that are complementary to each other.

10. The label according to one of the preceding claims, wherein the at least one first labelling moiety (108) and/or the at least one second labelling moiety (112) is optically detectable.

11. The label according to one of the preceding claims, wherein the at least one first labelling moiety (108) and/or the at least one second labelling moiety (112) have identical optical characteristics,
or wherein the at least one first labelling moiety (108) and/or the at least one second labelling moiety (112) have different optical characteristics.

12. The label according to one of the preceding claims, wherein the at least one first labelling moiety (108) and the at least one second labelling moiety (112) are configured for non-radiative energy transfer between them.

13. The label according to one of the preceding claims, wherein the at least one first labelling moiety (108) and the at least one second labelling moiety (112) are both attached to either the first nucleic acid strand (106) or the second nucleic acid strand (110).

14. The label according to one of the preceding claims 1 to 12, wherein the at least one first labelling moiety (108) is attached to the first nucleic acid strand (106).

15. The label according to one of the preceding claims, wherein the first nucleic acid strand (106) extends along a first direction and a plurality of the first labelling moieties (108) are arranged on the first nucleic acid strand (106) along the first direction, and/or the second nucleic acid strand (110) extends along a second direction and a plurality of the second labelling moieties (112) are arranged on the second nucleic acid strand (110) along the second direction.

16. The label according to one of the preceding claims, comprising a third label part comprising a third nucleic acid strand (708), wherein the third nucleic acid strand (708) is configured to form a triplex with the first nucleic acid strand (106) and the second nucleic acid strand (110).

17. The label according to one of the preceding claims, wherein each labelling moiety (108, 112) is equally spaced from any adjacent labelling moiety (108, 112).

18. A marker (100, 500, 600, 700, 1000) for analysing a biological sample with a plurality of target analytes (116, 120) comprising:
a label according to one of the preceding claims comprising a first label part and a second label part,
a first marker part (102) comprising a first affinity reagent (114) and the first label part, and
a second marker part (104) comprising a second affinity reagent (118) and the second label part, and
wherein the first affinity reagent (114) and the second affinity reagent (118) are each configured to bind specifically to one of the target analytes (116, 120) of the biological sample.

19. A method for analysing a biological sample, the method comprising the following steps:
introducing at least one marker (100, 500, 600, 700, 1000) according to claim 18 into the biological sample,
optionally generating a first optical readout of the biological sample with the marker (100, 500, 600, 700, 1000),
removing the blocking nucleic acid strand (126, 128) from the marker (100, 500, 600, 700, 1000), and
generating an optical readout or a further optical readout of the biological sample with the marker (100, 500, 600, 700, 1000), and
optionally calculating a fold change between the first and second optical readout or comparing the first optical readout and the further optical readout.
